# EUROPÄISCHE PATENTANMELDUNG

(11) **EP 0 642 022 A2**
(43) Veröffentlichungstag der Anmeldung: **08.03.1995**
(21) Anmeldenummer: 94113692.1
(22) Anmeldetag: 01.09.1994
(51) Int. Cl.: G01N 33/569, G01N 33/96, G01N 33/50

(54) **Mittel zur Stabilisierung von Blut**

(30) Priorität: 07.09.1993 DE 4330213
(71) Anmelder: Patscheke, Heinrich, Prof. Dr., D-76133 Karlsruhe (DE); Ruf, Andreas, Dr.med., D-76356 Weingarten (DE)
(72) Erfinder: Patscheke, Heinrich, Prof. Dr., D-76133 Karlsruhe (DE); Ruf, Andreas, Dr.med., D-76356 Weingarten (DE)
(74) Vertreter: Ratzel, Gerhard, Dr.

(57) **Zusammenfassung**

Die vorliegende Erfindung betrifft ein Mittel zur Stabilisierung von Blut, welches aus einer wässrigen Lösung von Methacrolein und Formaldehyd besteht.

## Beschreibung

Die Erfindung betrifft ein Mittel zur Stabilisierung insbesondere von menschlichem Vollblut und Blutzell-Präparaten, vorzugsweise für die immunologische Bestimmung von Blutzell-Antigenen mit fluoreszenzmarkierten Antikörpern.

Zu den klassischen Blutzellantigenen gehören diejenigen Blutgruppen-Substanzen, bei deren Bestimmung nach wie vor herkömmliche Agglutinationsmethoden dominieren. Dagegen erfolgt die Bestimmung von Antigenen der Granulozyten, Lymphozyten, Monozyten und Thrombozyten heute weitgehend mittels fluoreszenzmarkierter Antikörper. Durch ihre immunologischen Eigenschaften werden Blutzellen nicht nur eindeutig den einzelnen Zellklassen zugeordnet, sondern auch ihr Funktionszustand charakterisiert. In der medizinischen Diagnostik haben daher immunzytometrische Verfahren eine weite Verbreitung erfahren. Sie dienen derzeit insbesondere der Unterscheidung von Leukämieformen, der Lymphozyten-Differenzierung und der Erkennung und Beurteilung von Immundefekten.

Zur Erkennung bestimmter antigener Epitope werden monoklonale Antikörper eingesetzt, die mit unterschiedlichen Fluorochromen markiert sind. Mit einem Fluoreszenzmikroskop oder dem Flowzytometer lassen sie sich auf einzelnen Zellen direkt nachweisen, wo sie dann die Präsenz bestimmter Antigene anzeigen. Von einer indirekten Markierung spricht man, wenn nicht der primäre, das fragliche Epitop erkennende Antikörper fluoreszenzmarkiert ist, sondern ein zweiter, der gegen das Protein des primären Antikörpers gerichtet ist und von diesem gebunden wird. Die Immuntypisierung von Blutzellen mit fluoreszierenden Antikörpern stützt sich heute insbesondere auf die Flowzytometrie, die es erlaubt, in weniger als 1 Minute Tausende von Einzelzellen zu untersuchen und von jeder Zelle neben ihren Fluoreszenzeigenschaften die charakteristischen Streulichteigenschaften zu erfassen. Während die Fluoreszenzmessung zur Quantifizierung zellgebundener Antikörper dient, reflektieren die Streulichteigenschaften morphologische Merkmale der einzelnen Zellklassen. Mit der Flowzytometrie sind daher statistisch gut abgesicherte Resultate zu erzielen, deren Richtigkeit auf der teilautomatisierten, gleichzeitigen Auswertung mehrerer zellulärer Merkmale beruht.

Seit man die leistungsfähige Methode der Flowzytometrie in der medizinischen Diagnostik einsetzt, ist zunehmend deutlich geworden, daß immunzytometrische Resultate erheblich vom Funktionszustand der untersuchten Zellen abhängen. Die Expression der untersuchten Antigene hängt nämlich in hohem Maße vom Aktivierungszustand der Zellen und Einflüssen aufgrund von Handhabung und Aufbewahrung der Blut-Probe bzw. des Zellpräparates ab. So werden beispielsweise Antigene nach wenigen Stunden durch zelluläre Proteasen zerstört. Durch solche präanalytischen Einflüsse werden die Resultate qualitativ und quantitativ beeinflußt. Bei sehr rasch reagierenden Zellen wie den Thrombozyten, sind dramatische Veränderungen verschiedener Oberflächen/Antigene unvermeidlich, wenn nicht unmittelbar bei der Probennahme eine Stabilisierung der Zellen vorgenommen wird. Besonders kritisch sind diese Störeinflüsse dann, wenn vor der Messung eine Aufarbeitung oder Isolierung der Blutzellen erforderlich ist.

Für die immunologische Phänotypisierung von Blutzellen, insbesondere aber für quantitative Untersuchungen der Antigen-Expression, wäre es daher von erheblichem Nutzen, wenn man die Probe so stabilisieren könnte, daß präanalytische Veränderungen der zu untersuchenden Zell-Antigene ausgeschlossen werden. Ein solches Vorgehen ist unverzichtbar für die meisten Fragestellungen bei Thrombozyten und notwendig für Quantifizierungen der Antigenexpression von Granulozyten, Monozyten, Lymphozyten und Erythrozyten. Das gilt für die letztgenannten Zellen insbesondere dann, wenn die Proben nicht sofort nach der Probennahme untersucht werden können oder sollen.

Die Anforderungen, die ein geeignetes Stabilisierungsverfahren erfüllen sollte, also die hier vorliegenden sog. technischen Problemstellungen, lassen sich wie folgt definieren:
1. Mit dem Stabilisierungsmittel sollte eine Unterbrechung des Zellstoffwechsels so rasch eintreten, daß ein gleichzeitig zugegebener chemischer Zell-Stimulus unwirksam bleibt.
2. Dabei dürfen die immunologischen Eigenschaften der Zellen im Grundsatz nicht verändert werden, insbesondere sollte keine Antigendestruktion eintreten.
3. Die morphologischen Eigenschaften der Zellen sollten erhalten bleiben.
4. Die Autofluoreszenz der Zellen sollte nicht zunehmen.
5. Die Probe darf nicht gelieren, so daß sie weiterhin direkt der flowzytometrischen Analyse zugeführt werden kann.
6. Die Stabilität der Proben sollte mindestens 24 Stunden bei Raumtemperatur gewährleistet sein.

### Bedingungen 1 bis 6

Ein Verfahren, das diese Kriterien erfüllt, ist bislang nicht bekannt. Für Blutzellen, ausgenommen Thrombozyten, verzichtet man bisher auf eine Proben-Stabilisierung. Im Zuge der nationalen und internationalen Standardisierungsbemühungen wird vorussichtlich aber auch für andere Blutzellen neben den Thrombozyten die Frage nach Stabilisierungsverfahren unabweisbar werden. Für die Thrombozyten-Stabilisierung sind bisher Verfahren im Gebrauch, die unterschiedliche Teilaspekte der oben genannten Anforderungen erfüllen. Ein Verfahren beläßt es bei einer Antikoagulation mit Citrat (SHATTIL et al., 1987, GINSBERG et al., 1990), andere benutzen Formaldehyd (GEORGE et al., 1986, TSCHÖPE 1992) oder Glutaraldehyd (NIEUWENHUIS et al., 1987; RUF und PATSCHEKE, 1993) zur Zellfixierung.

Mit einem Antikoagulanz allein (SHATTIL et al.) ist es unmöglich, den Aktivierungsstoffwechsel definitiv zu unterbinden. Eine sichere Stabilisierung der Zelleigenschaften ist nicht möglich, so daß schon die Anfangsbedingung Nr. 1 nicht erfüllt wird. Als Konsequenz wird auch die Bedingung Nr. 3, nämlich die morphologische Fixierung, verfehlt. Andererseits erfüllt ein solches Vorgehen die Bedingungen 2, 4 und 5. So ist eine flowzytometrische Untersuchung der Thrombozyten möglich, allerdings nur unter streng kontrollierten Bedingungen. Solche sind gegebenenfalls in Modellversuchen in vitro einzuhalten, nicht aber mit hinreichender Sicherheit bei der Untersuchung von Patientenproben.

Andere Verfahren benutzen Formaldehyd (GEORGE et al 1g % Formaldehyd ; TSCHÖPE: DIII/Protokoll mit 0,5 g% Formaldehyd) als entscheidende Wirkkomponente.

Formaldehyd ist ein bewährtes Fixans für morphologische Untersuchungen. Es ist seit langem bekannt, daß Formaldehyd eine sehr rasche und effektive Unterbrechung des Zellstoffwechsels auch bei Thrombozyten sicherstellt. Auf Formaldehyd gestützte Verfahren erfüllen zwar die Voraussetzung Nr. 1. Ihr entscheidender Nachteil ist allerdings, daß Formaldehyd zu einer Antigendestruktion führt, die auch durch Neutralisieren des Formaldehyds nach kurzer Einwirkungszeit nicht aufzuhalten ist. Dies gilt an Thrombozyten beispielsweise für die wichtigen Antigene GMP-140 = CD 62, CD 41a, CD 42a. Damit scheidet Formaldehyd als geeignetes Reagenz zur generellen Stabilisierung für die Immunzytometrie aus. Andere Nachteile des Formaldehyds fallen daneben nicht ins Gewicht. So bleibt es unerheblich, daß Formaldehyd auch die Morphologie nicht sicher stabilisiert und zu einer Zunahme der Autofluoreszenz führt. Eignen könnte sich ein Stabilisierungsmittel mit Formaldehyd als alleiniger Wirtskomponente lediglich für solche Antigene, die Formaldehyd-unempfindlich sind. Die von verschiedenen Forschern an Thrombozyten getesteten, oben genannten Antigene, sind es nicht.

Ein anderes in der Morphologie bewährtes Fixationsmittel ist Glutaraldehyd. In niedrigen Konzentrationen von ca. 0,05 g% ist damit eine hinreichende Stabilisierung zu erreichen, ohne daß verschiedene thrombozytäre Antigene zerstört werden (NIEUWENHUIS et al.; RUF and PATSCHEKE, 1993). Ein wesentlicher Nachteil ist jedoch, daß Glutaraldehyd die Autofluoreszenz der Thrombozyten und mehr noch anderer Blutzellen erhöht. Die Zunahme der unspezifischen Autofluoreszenz kann dann die spezifische Fluoreszenz durch gebundene Antikörper überdecken und eine Auswertung einer geringen Antikörperbindung unmöglich machen. Das gilt insbesondere dann, wenn mit direkt markierten, primären Antikörpern gearbeitet wird, bei denen eine Amplifikation des Fluoreszenzsignals, wie es mit indirekten Markierungsmethoden möglich ist, entfällt. Damit werden vor allem Untersuchungen in Vollblut und ungereinigten Zellpräparaten stark eingeschränkt, bei denen hauptsächlich direkte Markierungsmethoden in Frage kommen, um Interferenzen von Plasma-Immunglobulinen zu vermeiden.

Die bisherigen Versuche, geeignete Mittel für ein Stabilisierungsverfahren zu finden, zeigen, daß das Kernproblem darin liegt, ein Mittel zu finden, das wirksam genug ist, um die Zellaktivität und die Zellmorphologie rasch und sicher zu stabilisieren, das aber gleichzeitig die Antigene nicht zerstört. Um die erste Bedingung zu erreichen, müssen die den Zellstoffwechsel steuernden Proteine einschließlich der Enzyme inaktiviert werden. Aber auch Antigene haben Proteinnatur oder sind Protein-Komponenten und sollten dabei unverändert bleiben. Die Schwierigkeit, das eine zu erreichen ohne das andere mit in Kauf nehmen zu müssen, kann daher nicht überraschen. Die Spezifität des erstrebten Stabilisierungsverfahrens liegt demgemäß offenbar darin, Proteine funktionell zu inaktivieren ohne ihre antigenen Eigenschaften zu beeinträchtigen.

Folgende Literaturstellen sind in vorangehenden Text zitiert:
1. Shattil S.J., Cunningham M., and Hoxie J.A: Detection of activated platelets in whole blood using activation-dependent monoclonal antibodies und flow cytometry. Blood 70: 307/315 (1987)
2. Ginsberg M.H., Frelinger A.L., Lam S.C.-T., Forsyth J., MxMillan R., Plow E.F., and Shattil S.J.: Analysis of platelet aggregation disorders based on flow cytometric analysis of membrane glycoprotein IIb/IIIa with conformation-specific monoclonal antibodies. Blod 76: 2017-2023 (1990)
3. George J.N., Pickett E.B., Saucerman S., McEver R.P., Kunicki T.J., Kieffer N. and Newman P.J.: Platelet surface glycoproteins. Studies on resting and activated platelets und platelet membrane microparticles in normal subjects, and observations in patients during adult respiratory distress syndrome and cardiac surgery. J. Clin. Invest. 78: 340-348 (1986)
4. Tschöpe D.: Markeanalyse und Funktionstets an Thrombozyten. In: Anwendungen der Durchflußzytometrie in der Laboratoriumsdiagnostik, 2. Regensburger Kurs, Sept. 1992, Universität Regensburg, Institut für Klinische Chemie und Laboratoriumsmedizin.
5. Nieuwenhuis H.K., van Oosterhout I.I.G., Rorieonuke E., van Iwaarden F., and Sixma J.J.: Studies with a monoclonal antibody against activated platelets: evidence that a secreted MW 53.000 lysosome-like-granule protein is exposed on the surface of activated platelets in the circulation. Blood 70: 838/845.
6. Ruf A. and Patscheke H.: Whole blood procedure for flow cytometric determination of platelet antigens. In: R.E. Scharf, K.J. Clemetson (Eds) Flow Cytometry of the Megakaryocte-Platelet System. Elsevier, Amsterdam 1993.

Die vorliegende Erfindung schafft ein Mittel zur Stabilisierung von Blut, das bei seiner Anwendung in der Lage ist, die oben genannten 6 Bedingungen für eine präanalytische Probenstabilisierung zu erfüllen.

Das erfindungsgemäße Mittel ist dadurch gekennzeichnet, daß es aus einer wässrigen Lösung von Methycrolein und Formaldehyd besteht. Bevorzugte Ausführungsformen sind in den Unteransprüchen offenbart.

Ein Vorgehen, nach dem die aufgeführten Resultate mit dem erfindungsgemäßen Mittel erreicht werden, ist das folgende:
1. Probenvorbereitung:
   Stabilisierung
   Blut wird
   a) aus einer gestauten oder ungestauten Cubitalvene abgenommen und mittels eines Butterfly's (Innendurchmesser 0,6 mm)
   b) direkt in das erfindungs gemäße Mittel überführt. Mischungsverhältnis ca. 1 : 1; Gesamtvolumen z.B. 600 µl.
      Inkubation für 5 Min. bei Raumtemperatur.
   Immunmarkierung
   5 µl der stabilisierten Blutprobe werden zu 45 µl Phosphatpuffer gegeben. Zugabe primärer Antikörper in sättigender Konzentration. Volumen ≦ 10 µl Inkubation für 15 Min. bei Raumtemperatur.
   direkt:
   Zugabe von 450 µl Phosphatpuffer.
   indirekt:
   Zugabe von 150 µl Phosphatpuffer.
   Zugabe sekundärer Fluorochrom- gekoppelter F(ab') - Fragmente in sättigender Konzentration: Volumen ≦ 20 µl.
   Inkubation 20 Min. bei RT.
   Zugabe von 300 µl Phosphatpuffer.
2. Messung:
   Flowzytometrische Bestimmung nach üblichem Vorgehen.
   a) Anstelle von Vollblut können in gleicher Weise Blutzellpräparate stabilisiert werden, z.B. mononukleare Zellen und Granulozyten, die nach Gradientenzentrifugation angereichert wurden, gewaschene u. gelfiltrierte Thrombozyten, plättchenreiches Plasma, Plättchenhochkonzentrate, Erythrozytenkonzentrate,
      Zellfraktionen nach Plasmapherese einschließlich Stammzellpräparaten. Gleiches gilt für Vollblut nach Lyse-Behandlung zur Erythozyten-Zerstörung vor einer Untersuchung der Leukozytenfraktion.
   b) Die Mischung der Probe mit dem erfindungsgemäßen Mittel kann auch mit anderen Geräten wie z.B. mit Pipetten oder Spritzen erfolgen.

Es folgt eine nähere Charakterisierung der Eigenschaften des erfindungsgemäßen Blut-Stabilisierungsmittels.

Aus den oben genannten Gründen stellen Thrombozyten die höchsten Anforderungen an das Fixationsmittel. Sie reagieren besonders rasch auf zahlreiche chemische und physikalische Reize mit der Exposition von Neoantigenen bzw. Veränderungen des Antigenmusters an ihrer Oberfläche. Sie machen dabei einen Formwandel von der Scheiben- in eine sphäroide Form mit Pseudopodien durch, der leicht zu quantifizieren ist (MILTON and FROJMOVIC, 1979). Sie stellen als kleinste Blutzellen auch besonders hohe Anforderungen an die Empfindlichkeit und Qualität des Meßverfahrens. Deshalb wird hier die Leistungsfähigkeit der vorliegenden Erfindung, insbesondere an Thrombozyten, dokumentiert.

Im einzelnen wurden die Eigenschaften des Anwendungsverfahrens wie folgt geprüft:

### Zu Bedingung 1:

Der Formwandel der Thrombozyten ist eine der empfindlichsten Reaktionen der Thrombozyten auf eine Stimulation, wobei aus Diskozyten Sphäroechinozyten werden. Er läßt sich mit einem mikroskopischen Verfahren nachweisen (MILTON and FROJMOVIC; 1979) und ist zur Überprüfung der sicheren und raschen Unterbrechung der Zellaktivität besonders geeignet. Der Formwandel sollte selbst dann ausbleiben, wenn das Stabilisierungsreagenz gleichzeitig mit einem starken Stimulus wie dem Thromboxan-Mimetikum U 46619 1 µmol/l oder ADP 10 µmol/l zugegeben wird.

Erstrecht sollte eine Antwort der Thrombozyten ausbleiben, wenn das Stabilisierungsreagenz vor dem Simulus zugesetzt wurde, und nachträglich zugesetzt, sollte das Stabilisierungsreagenz die Reaktion zeitgenau anhalten. Alle diese Bedingungen werden von dem angegebenen Anwendungsverfahren des erfindungsgemäßen Mittels erfüllt.

### Zu Bedingung 2:

Die Antigen-Expression von CD 61, CD 41a, CD 42a, CD 62, und CD 63 (monoklonale Antikörper von der Fa. DIANOVA; Hamburg und der Fa. Becton Dickinson; Heidelberg) wurde an nicht-stabilisierten Thrombozyten und solchen zu unterschiedlichen Zeitpunkten nach der Stabilisierung mit dem erfindungsgemäßen Mittel gemessen. Die Thrombozyten waren stimuliert oder unstimuliert. Eine Antigendestruktion wurde nicht beobachtet.
Mit unstimulierten neutrophilen Granulozyten, Monozyten und Lymphozyten wurden entsprechende Versuche für folgende Antigene duchgeführt: CD 14 für Monozyten, CD 3, CD 4, CD 8 für Lymphozyten. Die Zellen wurden nur unstimuliert eingesetzt. Eine Veränderung der Antigen-Expression wurde nicht beobachtet.

### Zu Bedingung 3:

Von den mit Formaldehyd fixierten Thrombozyten ist bekannt, daß sie nach der Fixierung eine Membranvesikulation an ihrer Oberfläche aufweisen (siehe unter 2.). Sie ist im Interferenzkontrast- oder Phasenkonstrast-Mikroskop gut zu erkennen und nimmt mit der Zeit zu. Sie beeinflußt die rheooptischen Eigenschaften der Thrombozyten und damit das Streulichtmuster im Flowzytometer. In den mit dem erfindungsgemäßen Mittel stabilisierten Thrombozyten bleibt diese Veränderung aus.

### Zu Bedingung 4:

Entscheidender Nachteil einer Fixierung mit Glutaraldehyd ist die damit verbundene Zunahme der Autofluoreszenz, insbesondere von Leukozyten und Thrombozyten. Sie macht die flowzytometrische Messung einer schwachen Antigen-Expression unmöglich (siehe unter 2.). Das erfindungsgemäße Mittel steigert die Autofluoreszenz weder der Thrombozyten noch Leukozyten in störendem Maße.

### Zu Bedingung 5:

Glutaraldehyd führt oberhalb einer Endkonzentration von ca. 0,5 g% in Vollblutproben leicht zu einer Gelierung durch Vernetzung der Plasmaproteine. Solche Proben sind insbesondere für flowzytometrische Untersuchungen unbrauchbar. Das erfindungsgemäße Mittel hat diesen Effekt nicht.

### Zu Bedingung 6:

Alle oben genannten Kriterien wurden für eine Aufbewahrungszeit von mindestens 24 Stunden in allen, mit dem erfindunsgemäßen Mittel stabilisierten Proben erfüllt.

Als Literaturstelle wird in diesem Zusammenhang genannt:
Milton J.G. and Frojmovic M.M.: Shape-changing agents produce abnormally large platelets in a hereditary "giant platelets syndrome (MPS)". J.Lab. Clin.Med. 93: 154-161 (1979)
Zusammenfassend ist folgendes festzustellen:
Es werden erfindungsgemäß Methylacrolein- sowie Formaldehyd enthaltende Lösungen geschaffen, die nach ihrem Vermischen mit Vollblut des Menschen eine Stabilisierung der darin enthaltenen Blutzellen, insbesondere Erythrozyten, Thrombozyten, Granulozyten, Lymphozyten einschließlich ihrer Subklassen und unreifen Vorstufen herbeiführen, wobei folgende Kriterien erfüllt werden:
a) Sofortige Unterbrechung der zellulären Stoffwechselaktivität.
   Nachzuweisen dadurch, daß native, diskoide Thrombozyten auf ADP 10 µmol/l nicht wie in der Kontrolle mit einem Formwandel reagieren, wenn gleichzeitig mit dem ADP die Stabilisierungslösung zugesetzt wird.
b) Keine Destruktion der Zell-Antigene.
   Nachzuweisen mit 3 oder mehr monoklonalen Antikörpern, die eine quantitativ gleiche Antigen-Expression in der unfixierten Probe und der fixierten Probe aufzeigen.
c) Morphologische Fixierung der Zellen.
   Nachzuweisen dadurch, daß stabilisierte Thrombozyten im Interferenz- oder Phasenkontrastmikroskop keine Membranvesikel an ihrer Oberfläche ausbilden. Als Positiv-Kontrolle zur Darstellung der Membranvesikel dienen Thrombozyten, die mit Formaldehyd in der Endkonzentration 1 g% fixiert sind und die Vesikulation in ihrer Mehrzahl zeigen.
d) Keine störende Zunahme der Autofluoreszenz der Zellen.
   Nachzuweisen in einem Flowzytometer mit Argon-Laser für die üblicherweise gemessene Grün- und Rot-Fluoreszenz.
e) Kein Gelieren der Probe.
f) Die Kriterien b) - e) werden auch nach 24 Stunden bewahren der Probe bei Raumtemperatur noch eingehalten.

Das erfindungsgemäße Mittel zur Stabilisierung von Blut ist auf Vollblut und auf andere Blutzellpräparate anwendbar, insbesondere auf mononukleäre Zellen und Granulozyten, die nach Gradientenzentrifugation angereichert wurden, Vollblut nach Lyse-Behandlung zur Erythrozyten-Zerstörung, gewaschene und gelfiltrierte Thrombozyten, plättchenreiches Plasma, Plättchenhochkonzentrate, Erythrozytenkonzentrate` Zellfraktionen nach Plasmapherese einschließlich Stammzellpräparaten.

Ferner können anstelle von Vollblut mit dem erfindungsgemäßen Mittel auch kultivierte Zellen stabilisiert werden, beispielsweise Zellen permanenter Zellinien wie HL-60 Zellen, U-937, Hep-G2-Zellen, Zellen von primären Zellkulturen wie Fibroblasten, Endothelzellen aus Nabelvenen, Zellen von primären Zellkulturen.

Schließlich können mit dem erfindungsgemäßen Mittel anstelle von Vollblut auch Zellfragmente wie beispielsweise Mikrosomen und Mikropartikel stabilisiert werden.

## Patentansprüche

1. Mittel zur Stabilisierung von Blut,
dadurch gekennzeichnet,
daß es aus einer wässrigen Lösung von Methacrolein und Formaldehyd besteht.

2. Mittel zur Stabilisierung von Blut nach Anspruch 1,
dadurch gekennzeichnet,
daß das Methacrolein in einem Konzentrationsbereich von 0,01 - 0,5 g% und das Formaldehyd in einem Konzentrationsbereich von 0,01 - 0,1 g%, beide gelöst in wässrigen Pufferlösungen von pH- 6,5 - 7,5 mit einer Osmolalität von 200 - 600 mosmol/kg vorliegen.

3. Mittel zur Stabilisierung von Blut nach Anspruch 1-2,
dadurch gekennzeichnet,
daß es einen Phosphatpuffer und/oder einen Piperazinpuffer und/oder einen Acetatpuffer enthält.

4. Mittel zur Stabilisierung von Blut nach Anspruch 1-3,
dadurch gekennzeichnet, daß es weitere Aldehyde und/oder Dialdehyde gelöst in Pufferlösungen enthält.
